(19) Europäisches Patentamt
European Patent Office
Office européen des brevets

(11) EP 0 657 172 B1

(12) EUROPÄISCHE PATENTSCHRIFT

(45) Veröffentlichungstag und Bekanntmachung des Hinweises auf die Patenterteilung:
**12.05.1999 Patentblatt 1999/19**

(51) Int. Cl.[6]: **A61K 38/15**

(21) Anmeldenummer: **94118680.1**

(22) Anmeldetag: **28.11.1994**

# (54) Endoparasitizide Mittel auf Basis von offenkettigen Tetradepsipeptiden

Endoparasiticide drugs on a basis of open chain tetradepsipeptides

Remèdes endoparasiticides à base de tetradepsipeptides chaînes ouvertes

(84) Benannte Vertragsstaaten:
**BE CH DE ES FR GB IT LI NL**

(30) Priorität: **09.12.1993 DE 4341992**

(43) Veröffentlichungstag der Anmeldung:
**14.06.1995 Patentblatt 1995/24**

(73) Patentinhaber: **BAYER AG**
**51368 Leverkusen (DE)**

(72) Erfinder:
- **Scherkenbeck, Jürgen, Dr.**
  **D-42929 Wermelskirchen (DE)**
- **Jeschke, Peter, Dr.**
  **D-51373 Leverkusen (DE)**
- **Plant, Andrew, Dr.**
  **D-51519 Odenthal (DE)**
- **Harder, Achim, Dr.**
  **D-51109 Köln (DE)**
- **Mencke, Norbert, Dr.**
  **D-51381 Leverkusen (DE)**

(56) Entgegenhaltungen:
**EP-A- 0 626 375** **EP-A- 0 626 376**
**DE-A- 4 317 458**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Die vorliegende Erfindung betrifft die Verwendung von offenkettigen Tetradepsipeptiden zur Bekämpfung von Endoparasiten.

Offenkettige Tetradepsipeptide sind als Ausgangsstoffe für endoparasitizid wirksame cyclische Depsipeptide mit 28 Ringatomen Gegenstand vorgängiger Patentanmeldungen (Deutsche Patentanmeldung P 43 17 457.4; P 43 17 432.9; P 43 17 458.2, entsprechend DE-A-43 17 457; DE-A-43 17 432; DE-A-43 17 458).

Über eine Verwendung dieser Verbindungen gegen Endoparasiten ist nichts bekannt.

Es wurde nun gefunden, daß die offenkettigen Tetradepsipeptide der allgemeinen Formel (I)

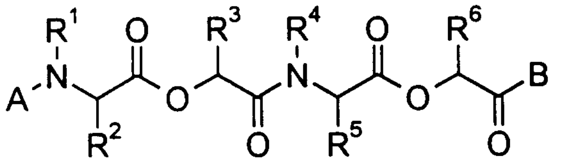

(I),

in welcher

A für Wasserstoff, Alkyl, Aralkyl oder einen Acylrest insbesondere der Formel -CO-$R^9$ steht, wobei

$R^9$ für geradkettiges oder verzweigtes Alkyl, Alkoxy, Aralkyl oder Aralkoxy mit bis zu 6 C-Atomen im Alkylteil steht,

$R^1$ und $R^4$ unabhängig voneinander für Wasserstoff, $C_1$-$C_8$-Alkyl, $C_3$-$C_6$-Cycloalkyl oder Aralkyl stehen,

$R^2$ und $R^5$ unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Aryl, Arylalkyl oder Hetarylmethyl, wobei als Substituenten genannt seien Halogen, Hydroxy, Alkyl, Alkoxy, Nitro oder ein Rest $NR^{10}R^{11}$, wobei $R^{10}$ und $R^{11}$ unabhängig voneinander für Wasserstoff oder Alkyl stehen oder $R^{10}$ und $R^{11}$ gemeinsam mit dem angrenzenden N-Atom für einen carbocyclischen 5-, 6-, 7- oder 8-gliedrigen Ring stehen, der gegebenenfalls auch durch O, S und N unterbrochen sein kann und gegebenenfalls durch $C_1$-$C_4$-Alkyl substituiert ist, stehen,

$R^3$ und $R^6$ unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Alkylthioalkyl, Alkylsulfinylalkyl, Alkysulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl sowie gegebenenfalls substituiertes Aryl, Arylalkyl oder Hetarylmethyl, wobei als Substituenten genannt seien Halogen, Hydroxy, Alkyl, Alkoxy, Nitro oder ein Rest $NR^{10}R^{11}$, wobei $R^{10}$ und $R^{11}$ unabhängig voneinander für Wasserstoff oder Alkyl stehen oder $R^{10}$ und $R^{11}$ gemeinsam mit dem angrenzenden N-Atom für einen carbocyclischen 5-, 6-, 7- oder 8-gliedrigen Ring stehen, der gegebenenfalls auch durch O, S und N unterbrochen sein kann und gegebenenfalls durch $C_1$-$C_4$-Alkyl substituiert ist, stehen,

B für Hydroxy, Alkoxy mit bis zu 4 Kohlenstoffatomen oder den Rest $NR^7R^8$ steht, wobei

$R^7$ und $R^8$ für Wasserstoff, Alkyl, Aralkyl oder Aryl stehen, sowie deren optische Isomere und Racemate, zur Bekämpfung von Endoparasiten in der Medizin und Tiermedizin Verwendung finden können.

Die erfindungsgemäß zu verwendenden offenkettigen Tetradepsipeptide sind durch die Formel (I) allgemein definiert. Vorzugsweise werden erfindungsgemäß diejenigen Verbindungen der Formel (I) verwendet, in welcher

A für Wasserstoff oder $C_{1-4}$-Alkyl oder Benzyl steht, oder für eine Gruppe der Formel -CO-$R^9$ steht, worin

$R^9$ für geradkettiges oder verzweigtes $C_{1-4}$-Alkyl, $C_{2-4}$-Alkenyl, $C_{1-4}$-Alkoxy oder Phenylalkoxy mit bis zu 6 Kohlenstoffatomen im Alkylteil, insbesondere für tert.-Butoxy, Benzyloxy, Ethoxy, Allyloxy, Fluorenyl-9-methoxy oder Methoxy steht,

$R^1$ und $R^4$ unabhängig voneinander für Wasserstoff, $C_{1-6}$-Alkyl, $C_{3-6}$-Cycloalkyl, Phenyl-$C_{1-4}$-alkyl stehen,

$R^2$ und $R^5$ unabhängig voneinander für Wasserstoff, geradkettiges oder verzweigtes $C_{1-8}$-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Octyl, Isooctyl, sec.-Octyl, Hydroxy-$C_1$-$C_6$-alkyl, insbesondere Hydroxymethyl, 1-Hydroxyethyl, $C_1$-$C_4$-Alkanoyloxy-$C_1$-$C_6$-alkyl, insbesondere Acetoxymethyl, 1-Acetoxyethyl, $C_1$-$C_4$-Alkoxy-$C_1$-$C_6$-alkyl, insbesondere Methoxymethyl, 1-Methoxyethyl, Aryl-$C_1$-$C_4$-alkyloxy-$C_1$-$C_6$-alkyl, insbesondere Benzyloxymethyl, 1-Benzyloxy-ethyl, Mercapto-$C_1$-$C_6$-alkyl, insbesondere Mercaptomethyl, $C_1$-$C_4$-Alkylthio-$C_1$-$C_6$-alkyl, insbesondere Methylthioethyl, $C_1$-$C_4$-Alkylsulfinyl-$C_1$-$C_6$-alkyl, insbesondere Methylsulfinylethyl, $C_1$-$C_4$-Alkylsulfonyl-$C_1$-$C_6$-alkyl, insbesondere Methylsulfonylethyl, Carboxy-$C_1$-$C_6$-alkyl, insbesondere Carboxymethyl, Carboxyethyl, $C_1$-$C_4$-Alkoxycarbonyl-$C_1$-$C_6$-alkyl, insbesondere Methoxycarbonylmethyl, Ethoxycarbonylethyl, $C_1$-$C_4$-Arylalkoxycarbonyl-$C_1$-$C_6$-alkyl, insbesondere Benzyloxycarbonylmethyl, Carbamoyl-$C_1$-$C_6$-alkyl, insbesondere Carbamoylmethyl, Carbamoylethyl, Amino-$C_1$-$C_6$-alkyl, insbesondere Aminopropyl, Aminobutyl, $C_1$-$C_4$-Alkylamino-$C_1$-$C_6$-alkyl, insbesondere Methylaminopropyl, Methylaminobutyl, $C_1$-$C_4$-Dialkylamino-$C_1$-$C_6$-alkyl, insbesondere Dimethylaminopropyl, Dimethylaminobutyl, Guanido-$C_1$-$C_6$-alkyl, insbesondere Guanidopropyl, $C_1$-$C_4$-Alkoxycarbonylamino-$C_1$-$C_6$-alkyl, insbesondere tert.-Butoxycarbonylaminopropyl, tert.-Butoxycarbonylaminobutyl, 9-Fluorenylmethoxycarbonyl(Fmoc)aminopropyl, 9-Fluorenylmethoxycarbonyl(Fmoc)aminobutyl, $C_2$-$C_8$-Alkenyl, insbesondere Vinyl, Allyl, Butenyl, $C_3$-$C_7$-Cycloalkyl, insbesondere Cyclopentyl, Cyclohexyl, Cycloheptyl, $C_3$-$C_7$-Cycloalkyl-$C_1$-$C_4$-alkyl, insbesondere Cyclopentylmethyl, Cyclohexylmethyl, Cycloheptylmethyl, Aryl, Phenyl-$C_1$-$C_4$-alkyl, insbesondere Phenylmethyl, das gegebenenfalls durch Reste aus der Reihe Halogen, insbesondere Fluor, Chlor, Brom oder Iod, Hydroxy, $C_1$-$C_4$-Alkoxy, insbesondere Methoxy oder Ethoxy, $C_1$-$C_4$-Alkyl, insbesondere Methyl, Nitro oder einen Rest $-NR^{10}R^{11}$ substituiert sein kann, wobei $R^{10}$ und $R^{11}$ unabhängig voneinander für Wasserstoff oder Alkyl stehen oder $R^{10}$ und $R^{11}$ gemeinsam mit dem angrenzenden N-Atom für einen carbocyclischen 5-, 6-, 7- oder 8-gliedrigen Ring stheen, der gegebenenfalls durch $C_1$-$C_4$-Alkyl substituiert ist, Hetarylmethyl, insbesondere Benzo[b]thien-2-yl-methyl, Benzo[b]thien-3-yl-methyl, Naphth-1-yl-methyl, Naphth-2-yl-methyl, Pyrid-2-yl-methyl, Pyrid-3-yl-methyl, Fur-2-yl-methyl, Fur-3-yl-methyl, Indol-3-yl-methyl, N-Methyl-indol-3-yl-methyl, imidazol-4-yl-methyl, N-Methyl-imidazol-4-yl-methyl, stehen,

$R^3$ und $R^6$ unabhängig voneinander für Wasserstoff, geradkettiges oder verzweigtes $C_1$-$C_8$-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Octyl, Isooctyl, sec.-Octyl, Hydroxy-$C_1$-$C_6$-alkyl, insbesondere Hydroxymethyl, 1-Hydroxyethyl, $C_1$-$C_4$-Alkanoyloxy-$C_1$-$C_6$-alkyl, insbesondere Acetoxymethyl, 1-Acetoxyethyl, $C_1$-$C_4$-Alkoxy-$C_1$-$C_6$-alkyl, insbesondere Methoxymethyl, 1-Methoxyethyl, Aryl-$C_1$-$C_4$-alkyloxy-$C_1$-$C_6$-alkyl, insbesondere Benzyloxymethyl, 1-Benzyloxyethyl, Mercapto-$C_1$-$C_6$-alkyl, insbesondere Mercaptomethyl, $C_1$-$C_4$-Alkylthio-$C_1$-$C_6$-alkyl, insbesondere Methylthioethyl, $C_1$-$C_4$-Alkylsulfinyl-$C_1$-$C_6$-alkyl, insbesondere Methylsulfinylethyl, $C_1$-$C_4$-Alkylsulfonyl-$C_1$-$C_6$-alkyl, insbesondere Methylsulfonylethyl, Carboxy-$C_1$-$C_6$-alkyl, insbesondere Carboxymethyl, Carboxyethyl, $C_1$-$C_4$-Alkoxycarbonyl-$C_1$-$C_6$-alkyl, insbesondere Methoxycarbonylmethyl, Ethoxycarbonylethyl, $C_1$-$C_4$-Arylalkoxycarbonyl-$C_1$-$C_6$-alkyl, insbesondere Benzyloxycarbonylmethyl, Carbamoyl-$C_1$-$C_6$-alkyl, insbesondere Carbamoylmethyl, Carbamoylethyl, Amino-$C_1$-$C_6$-alkyl, insbesondere Aminopropyl, Aminobutyl, $C_1$-$C_4$-Alkylamino-$C_1$-$C_6$-alkyl, insbesondere Methylaminopropyl, Methylaminobutyl, $C_1$-$C_4$-Dialkylamino-$C_1$-$C_6$-alkyl, insbesondere Dimethylaminopropyl, Dimethylaminobutyl, $C_2$-$C_8$-Alkenyl, insbesondere Vinyl, Allyl, Butenyl, $C_3$-$C_7$-Cycloalkyl, insbesondere Cyclopentyl, Cyclohexyl, Cycloheptyl, $C_3$-$C_7$-Cycloalkyl-$C_1$-$C_4$-alkyl, insbesondere Cyclopentylmethyl, Cyclohexylmethyl, Cycloheptylmethyl, Aryl, Phenyl-$C_1$-$C_4$-alkyl, insbesondere Phenylmethyl, das gegebenenfalls durch Reste aus der Reihe Halogen, insbesondere Fluor, Chlor, Brom oder Iod, Hydroxy, $C_1$-$C_4$-Alkoxy, insbesondere Methoxy oder Ethoxy, $C_1$-$C_4$-Alkyl, insbesondere Methyl, Nitro oder einen Rest $-NR^{10}R^{11}$ substituiert sein kann, wobei $R^{10}$ und $R^{11}$ unabhängig voneinander für Wasserstoff oder Alkyl stehen oder $R^{10}$ und $R^{11}$ gemeinsam mit dem angrenzenden N-Atom für einen carbocyclischen 5-, 6-, 7- oder 8-gliedrigen Ring stheen, der gegebenenfalls durch $C_1$-$C_4$-Alkyl substituiert ist, Hetarylmethyl, insbesondere Benzo[b]thien-2-yl-methyl, Benzo[b]thien-3-yl-methyl, Naphth-1-yl-methyl, Naphth-2-yl-methyl, Pyrid-2-yl-methyl, Pyrid-3-yl-methyl,

Fur-2-yl-methyl, Fur-3-yl-methyl, Indol-3-yl-methyl, N-Methyl-indol-3-yl-methyl, imidazol-4-yl-methyl, N-Methyl-imidazol-4-yl-methyl, stehen.

B für Hydroxy, tert.-Butoxy oder den Rest $NR^7R^8$ steht wobei

$R^7$ und $R^8$ unabhängig voneinander für Wasserstoff, geradkettiges oder verzweigtes $C_{1-6}$-Alkyl insbesondere Methyl, Ethyl, Isopropyl, tert-Butyl, Benzyl, Cyclopropyl, Cyclohexyl, Phenyl stehen sowie deren optische Isomere und Racemate.

Besonders bevorzugt werden erfindungsgemäß diejenigen Verbindungen der Formel (I) verwendet, in welcher

A für Wasserstoff, Benzyl oder eine Gruppe $COR^9$ steht,

$R^9$ für geradkettiges oder verzweigtes Alkoxy oder Arylalkoxy mit bis zu 6 Kohlenstoffatomen im Alkylteil, insbesondere für tert.-Butoxy, Benzyloxy steht,

$R^1$ und $R^4$ unabhängig voneinander für Wasserstoff, $C_1$-$C_4$-Alkyl, $C_3$-$C_6$-Cycloalkyl oder Benzyl stehen,

$R^2$ und $R^5$ unabhängig voneinander für Wasserstoff, geradkettiges oder verzweigtes $C_1$-$C_8$-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Hydroxy-$C_1$-$C_6$-alkyl, insbesondere Hydroxymethyl, 1-Hydroxyethyl, $C_1$-$C_4$-Alkanoyloxy-$C_1$-$C_6$-alkyl, insbesondere Acetoxymethyl, 1-Acetoxyethyl, $C_1$-$C_4$-Alkoxy-$C_1$-$C_6$-alkyl, insbesondere Methoxymethyl, 1-Methoxyethyl, Aryl-$C_1$-$C_4$-alkyloxy-$C_1$-$C_6$-alkyl, insbesondere Benzyloxymethyl, 1-Benzyloxyethyl, $C_1$-$C_4$-Alkoxycarbonylamino-$C_1$-$C_6$-alkyl, insbesondere tert.-Butoxycarbonylaminopropyl, tert.-Butoxycarbonylaminobutyl, $C_2$-$C_8$-Alkenyl, insbesondere Vinyl, Allyl, $C_3$-$C_7$-Cycloalkyl, insbesondere Cyclopentyl, Cyclohexyl, Cycloheptyl, $C_3$-$C_7$-Cycloalkyl-$C_1$-$C_4$-alkyl, insbesondere Cyclopentylmethyl, Cyclohexylmethyl, Cycloheptylmethyl, Phenyl, Pyridyl, Phenyl-$C_1$-$C_4$-alkyl, insbesondere Phenylmethyl, die gegebenenfalls durch einen oder mehrere gleiche oder verschiedene der oben angegebenen Reste substituiert sein können, stehen,

$R^3$ und $R^6$ unabhängig voneinander für Wasserstoff, geradkettiges oder verzweigtes $C_1$-$C_8$-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Octyl, Isooctyl, sec.-Octyl, Hydroxy-$C_1$-$C_6$-alkyl, insbesondere Hydroxymethyl, Aryl-$C_1$-$C_4$-alkyloxy-$C_1$-$C_6$-alkyl, insbesondere Benzyloxymethyl, 1-Benzyloxyethyl, Carboxy-$C_1$-$C_6$-alkyl, insbesondere Carboxymethyl, Carboxyethyl, $C_1$-$C_4$-Alkoxycarbonyl-$C_1$-$C_6$-alkyl, insbesondere Methoxycarbonylmethyl, Ethoxycarbonylethyl, $C_1$-$C_4$-Arylalkoxycarbonyl-$C_1$-$C_6$-alkyl, insbesondere Benzyloxycarbonylmethyl, $C_1$-$C_4$-Alkylamino-$C_1$-$C_6$-alkyl, insbesondere Methylaminopropyl, Methylaminobutyl, $C_1$-$C_4$-Dialkylamino-$C_1$-$C_6$-alkyl, insbesondere Dimethylaminopropyl, Dimethylaminobutyl, $C_2$-$C_8$-Alkenyl, insbesondere Vinyl, Allyl, Butenyl, $C_3$-$C_7$-Cycloalkyl, insbesondere Cyclopentyl, Cyclohexyl, Cycloheptyl, $C_3$-$C_7$-Cycloalkyl-$C_1$-$C_4$-alkyl, insbesondere Cyclopentylmethyl, Cyclohexylmethyl, Cycloheptylmethyl, Hetaryl, Phenyl, Pyridylmethyl, Phenyl-$C_1$-$C_4$-alkyl, insbesondere Phenylmethyl die gegebenenfalls durch einen oder mehrere gleiche oder verschiedene der oben angegebenen Reste substituiert sein können stehen,

B für Hydroxy oder tert.-Butoxy steht, sowie deren optische Isomere und Racemate.

Ganz besonders bevorzugt werden erfindungsgemäß diejenigen Verbindungen der Formel (I) verwendet, in welcher

A für Wasserstoff oder Benzyl steht,

$R^1$ und $R^4$ unabhängig voneinander für Wasserstoff, Methyl, Ethyl, Propyl und iso-Propyl stehen,

$R^2$ und $R^5$ unabhängig voneinander für Wasserstoff, geradkettiges oder verzweigtes $C_1$-$C_8$-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, $C_2$-$C_8$-Alkenyl, insbesondere Allyl, $C_3$-$C_7$-Cycloalkyl-$C_1$-$C_4$-alkyl, insbesondere Cyclohexylmethyl, Phenyl-$C_1$-$C_4$-alkyl, insbesondere Phe-

nylmethyl stehen,

$R^3$ und $R^6$ unabhängig voneinander für Wasserstoff, geradkettiges oder verzweigtes $C_1$-$C_8$-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, $C_2$-$C_8$-Alkenyl, insbesondere Vinyl, Allyl, $C_3$-$C_7$-Cycloalkyl-$C_1$-$C_4$-alkyl, insbesondere Cyclohexylmethyl, Pyridylmethyl, Phenyl-$C_1$-$C_4$-alkyl, insbesondere Phenylmethyl, die gegebenenfalls durch einen oder mehrere gleiche oder verschiedene der oben angegebenen Reste substituiert sein können,

B für Hydroxy oder tert.-Butoxy steht,
sowie deren optische Isomere und Racemate.

Im einzenen seien folgende Verbindungen der allgemeinen Formel (I) genannt, in welcher die Reste A, $R^1$ bis $R^6$ und B die in der folgenden Tabelle angegebenen Bedeutungen haben:

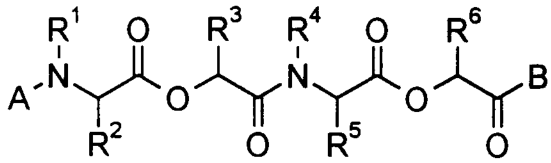

(I)

| Nr. | A | B | $R^1$ | $R^2$ | $R^3$ | $R^4$ | $R^5$ | $R^6$ |
|---|---|---|---|---|---|---|---|---|
| 1 | Bzl | $O^tBu$ | Me | $^iBu$ | Me | Me | $^iPr$ | Bzl |
| 2 | H | $O^tBu$ | Me | $^iBu$ | Me | Me | $^iPr$ | Bzl |
| 3 | Bzl | H | Me | $^iBu$ | Me | Me | $^iPr$ | Bzl |
| 4 | Bzl | $O^tBu$ | Me | $^iPr$ | Me | Me | $^iBu$ | Bzl |
| 5 | Bzl | $O^tBu$ | Me | $^sBu$ | Me | Me | $^sBu$ | Bzl |
| 6 | H | $O^tBu$ | Me | $^iPr$ | Me | Me | $^iPr$ | Bzl |
| 7 | H | $O^tBu$ | Me | $^sBu$ | Me | Me | $^sBu$ | Bzl |
| 8 | H | $O^tBu$ | Me | $^iBu$ | Me | Me | $^iBu$ | Bzl |
| 9 | Bzl | $O^tBu$ | Pr | $^iBu$ | Me | Pr | $^iBu$ | Bzl |
| 10 | Bzl | $O^tBu$ | Et | $^iBu$ | Me | Et | $^iBu$ | Bzl |
| 11 | Bzl | $O^tBu$ | $^iPr$ | $^iBu$ | Me | $^iPr$ | $^iBu$ | Bzl |
| 12 | H | $O^tBu$ | Pr | $^iBu$ | Me | Pr | $^iBu$ | Bzl |
| 13 | H | $O^tBu$ | Et | $^iBu$ | Me | Et | $^iBu$ | Bzl |
| 14 | Bzl | H | Pr | $^iBu$ | Me | Pr | $^iBu$ | Bzl |
| 15 | Bzl | H | Et | $^iBu$ | Me | Et | $^iBu$ | Bzl |
| 16 | Bzl | H | $^iPr$ | $^iBu$ | Me | $^iPr$ | $^iBu$ | Bzl |
| 17 | H | $O^tBu$ | $^iPr$ | $^iBu$ | Me | $^iPr$ | $^iBu$ | Bzl |
| 18 | Bzl | $O^tBu$ | Me | Bzl | Me | Me | Bzl | Bzl |
| 19 | H | $O^tBu$ | Me | Bzl | Me | Me | Bzl | Bzl |
| 20 | Bzl | H | Me | Bzl | Me | Me | Bzl | Bzl |
| 21 | H | H | Pr | $^iBu$ | Me | Pr | $^iBu$ | Bzl |
| 22 | Bzl | $O^tBu$ | Me | $^iBu$ | Me | Me | $^iBu$ | 2-Cl-Bzl |
| 23 | H | $O^tBu$ | Me | $^iBu$ | Me | Me | $^iBu$ | 2-Cl-Bzl |
| 24 | Bzl | H | Me | $^iBu$ | Me | Me | $^iBu$ | 2-Cl-Bzl |
| 25 | Bzl | $O^tBu$ | Me | Me | Me | Me | Me | Bzl |
| 26 | H | $O^tBu$ | Me | Me | Me | Me | Me | Bzl |
| 27 | Bzl | H | Me | Me | Me | Me | Me | Bzl |

| Nr. | A | B | $R^1$ | $R^2$ | $R^3$ | $R^4$ | $R^5$ | $R^6$ |
|---|---|---|---|---|---|---|---|---|
| 28 | Bzl | $O^tBu$ | Me | Pr | Me | Me | Pr | Bzl |
| 29 | Bzl | $O^tBu$ | Me | $^sBu$ | $^iPr$ | Me | Bzl | $^iPr$ |
| 30 | Bzl | $O^tBu$ | Me | $^sBu$ | $^iPr$ | Me | Bzl | $^iPr$ |
| 31 | Bzl | $O^tBu$ | Me | $^iBu$ | Me | Me | $^iBu$ | Me |
| 32 | H | $O^tBu$ | Me | $^iBu$ | Me | Me | $^iBu$ | Me |
| 33 | Bzl | $O^tBu$ | Me | $^sBu$ | Me | Me | $^sBu$ | Me |
| 34 | H | $O^tBu$ | Me | $^sBu$ | Me | Me | $^sBu$ | Me |
| 35 | H | $O^tBu$ | Me | n-Bu | Me | Me | Bu | Me |
| 36 | Bzl | H | Me | $^sBu$ | Me | Me | $^sBu$ | Me |
| 37 | Bzl | $O^tBu$ | Me | Pr | Me | Me | Pr | Me |
| 38 | Bzl | $O^tBu$ | Me | $^iPr$ | Me | Me | $^iPr$ | Me |
| 39 | Bzl | $O^tBu$ | Me | Bzl | Bzl | Me | Bzl | Me |
| 40 | Bzl | $O^tBu$ | Me | $^sBu$ | Bzl | Me | $^sBu$ | Me |
| 41 | Bzl | $O^tBu$ | Me | $^iBu$ | H | Me | $^iBu$ | H |
| 42 | H | $O^tBu$ | Me | $^iBu$ | Me | Me | $^iBu$ | Me |
| 43 | Bzl | H | Me | $^iBu$ | Me | Me | $^iBu$ | Me |
| 44 | Bzl | H | Me | $^iBu$ | H | Me | $^iBu$ | H |
| 45 | H | $O^tBu$ | Me | $^iBu$ | H | Me | $^iBu$ | H |
| 46 | H | H | Me | $^iBu$ | Me | Me | $^iBu$ | Me |
| 47 | H | H | Me | $^sBu$ | Me | Me | $^sBu$ | Me |
| 48 | H | H | Me | Pr | Me | Me | Pr | Me |

Bzl = Benzyl
Bu = Butyl
Me = Methyl
Pr = Propyl
Et = Ethyl

Die Verbindungen der allgemeinen Formel (I) können in optisch aktiven, stereoisomeren Formen oder als racemische Gemische vorliegen. Vorzugsweise werden jedoch die optisch aktiven Formen der Verbindungen der allgemeinen Formel (I) verwendet.

Die Herstellung der erfindungsgemäß zu verwendenden offenkettigen Tetradepsipeptide der allgemeinen Formel (I) ist in vorgängigen Patentanmeldungen beschrieben (vgl. Deutsche Patentanmeldungen P 43 17 457.4; P 43 17 432.9, P 43 17 458.2, entsprechend DE-A-43 17 457; DE-A-43 17 432; DE-A-43 17 458).

Man erhält Verbindungen der Formel (I)

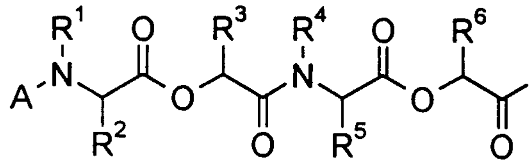

(I)

in welcher

A, $R^1$ bis $R^6$ und B die oben angegebene Bedeutung besitzen,

wenn man beispielsweise

Didepsipeptide der allgemeinen Formel (IIa)

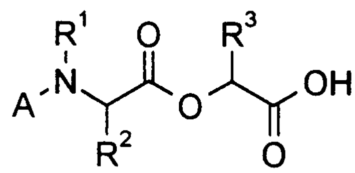

(IIa)

in welcher

A, $R^1$ bis $R^3$ die oben angegebene Bedeutung haben,

mit Didepsipeptiden der allgemeinen Formel (IIIa)

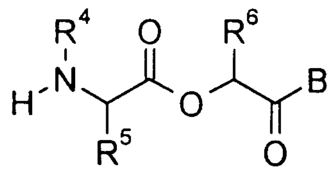

(IIIa)

in welcher

B, $R^4$ bis $R^6$ die weiter oben angegebene Bedeutung besitzen,

in Gegenwart geeigneter Kupplungsreagenzien, in Gegenwart eines basischen Reaktionshilfsmittels und in Gegenwart eines Verdünnungsmittels zu den Tetradepsipeptiden der allgemeinen Formel (I) umsetzt.

Die als Ausgangsverbindungen verwendeten, erfindungsgemäßen Didepsipeptide können nach klassischen Verfahren hergestellt werden, beispielsweise demjenigen, wie es von H.-G. Lerchen und H. Kunz (Tetrahedron Lett. 26 (43) (1958) S. 5257-5260; 28 (17) (1987) S. 1873-1876) unter Ausnutzung der Veresterungsmethode nach B.F. Gisin (Helv. Chim. Acta 56 (1973) S. 1476) beschrieben ist.

Die als Ausgangsmaterialien verwendeten N-Methyl-aminosäuren und 2-Halogencarbonsäurederivate sind teilweise bekannt (vgl. z.B. N-Methyl-aminosäuren: R. Bowmann et al., J. Chem. Soc. (1950), S. 1346; J.R. McDermott et al., Can. J. Chem. 51 (1973) S. 1915; H. Wurziger et al., Kontakte (Merck, Darmstadt) 3 (1987) S. 8; 2-Halogencarbonsäurederivate: S. M. Birnbaum et al., J. Amer. Chem. Soc. 76 (1954), S. 6054, C.S. Rondestvedt, Jr. Et al., Org. Reactions 11 (1960) S. 189 (Review)) bzw. können nach den dort beschriebenen Verfahren erhalten werden.

Für die Kupplungsreaktion der als Ausgangsverbindungen eingesetzten Didepsipeptide (IIa), (IIa) finden alle Kupplungsreagenzien, die zur Herstellung einer Amidbindung geeignet sind (vgl. z.B.: Houben-Weyl, Methoden der organischen Chemie, Band 15/2; Bodanszky et al., Peptide Synthesis 2nd ed. (Wiley & Sons, New York 1976) oder Gross, Meienhofer, The Peptides: Analysis synthesis, biology (AWcademic Press, New York 1979), Verwendung.

Die offenkettigen Tetradepsipeptide der Formel (I) können somit nach einer Reaktionssequenz erhalten werden, die die folgenden Stufen umfaßt:

a) Synthese der Didepsipeptide der Formeln (II) und (III):

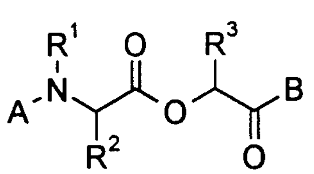

(II)

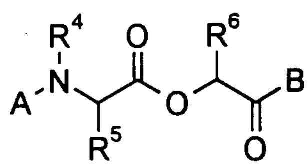

(III)

worin A eine N-terminale Schutzgruppe, wie z.B. die Benzyl oder Benzyloxycarbonylgruppe und B eine C-terminale Schutzgruppe, wie z.B. die tert.-Butoxygruppe, darstellt.

Dies entspricht beispielsweise für Formel (II) dem folgenden Reaktionsschema:

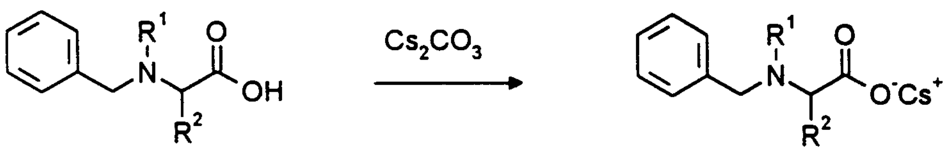


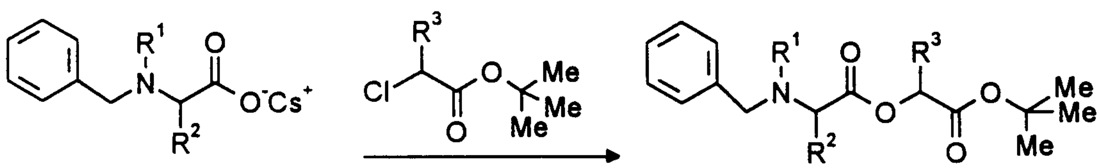

(IIb)

Die Herstellung der enantiomerenreinen Verbindungen der Formeln (II) und (III) kann gegebenenfalls auch über die Trennung der Diastereomere nach üblichen Methoden wie beispielsweise Kristallisation, durch Säulenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen.

Am Ende dieser Stufe kann entweder eine Entfernung der N-terminalen Schutzgruppe aus den Derivaten der Formel (III) in an sich bekannter Weise durchgeführt werden, beispielsweise durch katalytische Hydrierung, zur Herstellung der Derivate der Formel (IIIa) oder es kann in an sich bekannter Weise eine Abspaltung der C-terminalen Schutzgruppe aus den Derivaten der Formel (II), vorzugsweise durch Acidolyse, zur Darstellung der Derivate (IIa) erfolgen:

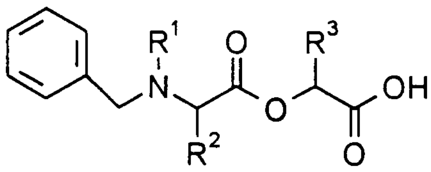

(IIa)

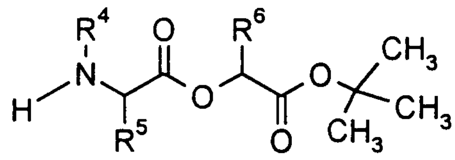

(IIIa)

b) Synthese des Tetradepsipeptids der Formel (I)

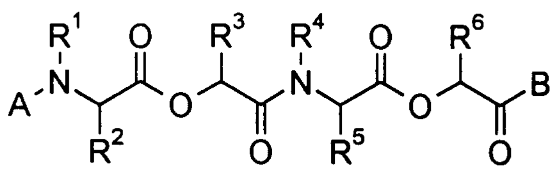

(I)

dies entspricht für Formel (I) dem nachfolgenden Reaktionsschema:

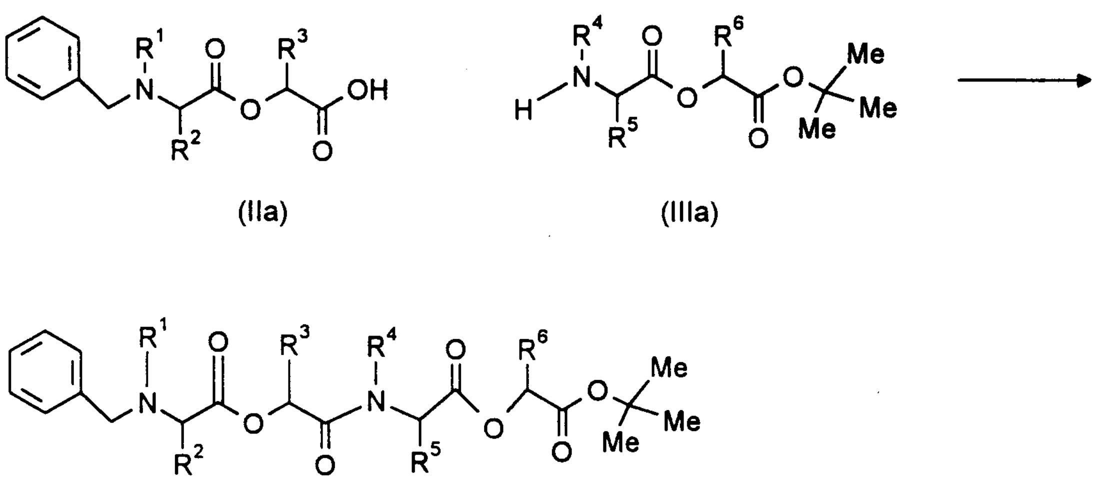

(IIa) (IIIa)

(Ia)

Man kann anschließend eine Entfernung der N-terminalen Schutzgruppe aus den Derivaten der Formel (Ia) durchführen, beispielsweise durch katalytische Hydrierung wie oben angegeben, zur Herstellung der Derivate der Formel (Ic), oder

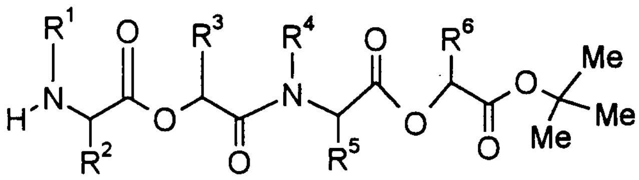

(Ic)

es kann in an sich bekannter Weise eine Abspaltung der C-terminalen Schutzgruppe, vorzugsweise durch Acidolyse, zur Darstellung der Derivate (Ib) erfolgen

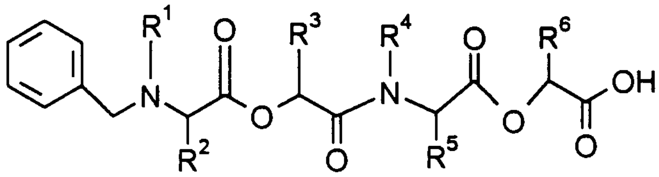

(Ib)

Die anfallenden Produkte lassen sich in üblicher Weise durch Umkristallisieren oder Säulenchromatographie reinigen (vgl. auch die Herstellungsbeispiele).

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zur Injektion; halbfeste Zubereitungen;

Formulierungen, bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulose-derivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel

werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl / Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleyl-alkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-β-iminodipropionat oder Lecithin;

anionaktive Tenside, wie Na-Laurylsulfat, Fettalkoholethersulfate, Mono/Dialkylpolyglykoletherorthophosphorsäureester-monoethynolaminsalz;

Als weitere Hilfsstoffe seien genannt: Viskositätserhöhende und die Emulsion stabilisierende Stoffe wie Carboxymethylcellulose, Methylcellulose und andere Cellulose-und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside ganannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

**Beispiel A**

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 10 |
| 2 | 10 |
| 3 | 10 |

**Herstellungsbeispiele**

**Beispiel 1**

**N-Ethyl-L-leucyl-D-lactyl-N-ethyl-L-leucyl-D-3-phenylmilchsäure-tert.-butylester**

Eine Lösung von Benzyl-L-EtLeu-D-Lac-L-EtLeu-D-PheLac-$O^tBu$ (7,83 g, 11,7 mmol) in Dioxan (50 ml) wurde mit Palladiumhydroxid (0,73 g, 20 %ig/Kohle) versetzt und bis zur Beendigung der Wasserstoffaufnahme bei Raumtemperatur und Normaldruck hydriert. Nach Filtration über Celite wurden 7,48 g (98 %) reines H-L-EtLeu-D-Lac-L-EtLeu-D-PheLac-$O^tBu$ erhalten.
FAB-MS m/z (%): 577 (62, (M+H) + ), 158 (100).

**Beispiel 2**

**N-Benzyl-N-propyl-L-leucyl-D-lactyl-N-propyl-L-leucyl-D-phenylmilchsäure**

In eine Lösung von Bzl-L-PrLeu-D-Lac-L-PrLeu-D-PheLac-$O^tBu$ (9,12 g, 13,2 mmol) in Dichlormethan (235 ml) wurde bei 0°C 2 Stunden HCl(g) eingeleitet und über Nacht bei Raumtemperatur nachgerührt. Anschließend wurde eingeengt und zweimal mit Dichlormethan codestilliert. Der Rückstand wurde in MeOH-$H_2O$ = 4:1 aufgenommen, mit 5,5 g basischem Ionenaustauscher versetzt und 2 Stunden gerührt. Nach Abfiltrieren des Ionenaustauschers und Einengen verblieben 8,1 g (96 %) Bzl-L-PrLeu-D-Lac-L-PrLeu-D-PheLac-OH.
FAB-MS m/z (%): 639 (32, M+H)+ ), 262 (92 %), 218 (108).

**Beispiel 3**

**N-Methyl-L-phenylalanyl-D-lactyl-N-methyl-D-phenylalanyl-D-milchsäure-tert.-butylester**

Eine Lösung von Bzl-L-MePhe-D-Lac-L-MePhe-D-PheLac-$O^tBu$ (3,2 g, 4,53 mmol) in Ethanol (20 ml) wird mit $Pd(OH)_2/C$ (20 %, 320 mg) versetzt und bei Raumtemperatur und Normaldruck bis zur Beendigung der Wasserstoffaufnahme hydriert. Nach Filtration über Celite werden 2,29 g (82 %) H-L-MePhe-D-Lac-L-MePhe-D-PheLac-$O^tBu$ erhalten.

**Beispiel 4**

**N-Benzyl-N-methyl-L-leucyl-D-lactyl-N-methyl-L-leucyl-D-3-phenylmilchsäure-tert.-butylester**

Eine auf 0°C gekühlte Lösung von H-L-MeLeu-D-PheLac-$O^tBu$ (8,0 g, 22,9 mmol) und Bzl-L-MeLeu-D-Lac-OH (8,44 g, 27,5 mmol) in Dichlormethan (80 ml) wurde mit Diisopropylethylamin (9,98 ml, 57,3 mmol) und Bis-(2-oxo-3-oxazolidinyl)-phosphorylchlorid (7,59 g, 29,8 mmol) versetzt, 1 Stunde bei 0°C und 1 Stunde bei Raumtemperatur gerührt. Nach Abfiltrieren des Niederschlages wurde die Lösung mit Dichlormethan verdünnt, dreimal mit wenig Wasser gewaschen, über Natriumsulfat getrocknet und eingeengt. Flash-Chromatographie an Kieselgel mit dem Laufmittel Cyclohexan-Ethylacetat = 15:1 lieferte 11,6 g (80 %) Bzl-L-MeLeu-D-Lac-L-MeLeu-D-PheLac-$O^tBu$.
FAB-MS m/z (%): 639 (37, (M+H)+ ), 190 (100).

Analog können die in der nachstehenden Tabelle 2 aufgeführten Verbindungen der allgemeinen Formel (I) als LDLD-Stereoisomere hergestellt werden.

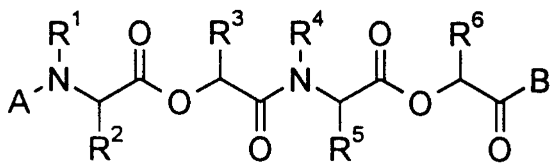

(I),

| Bsp. Nr. | A | B | $R^1$ | $R^2$ | $R^3$ | $R^4$ | $R^5$ | $R^6$ | Massenspektroskop. Daten |
|---|---|---|---|---|---|---|---|---|---|
| 5 | H | O$^t$Bu | Me | $^i$Bu | Me | Me | $^i$Pr | Bzl | 548 (1,$M^+$) |
| 6 | Bzl | H | Me | $^i$Bu | Me | Me | $^i$Pr | Bzl | 582 (0,5, $M^+$) |
| 7 | Bzl | O$^t$Bu | Me | $^i$Pr | Me | Me | $^i$Bu | Bzl | 611 (64, $(M+H)^+$) |
| 8 | Bzl | O$^t$Bu | Me | $^s$Bu | Me | Me | $^s$Bu | Bzl | 639 (92, $(M+H)^+$) |
| 9 | H | O$^t$Bu | Me | $^i$Pr | Me | Me | $^i$Pr | Bzl | 521 (100, $(M+H)^+$) |
| 10 | H | O$^t$Bu | Me | $^s$Bu | Me | Me | $^s$Bu | Bzl | 549 (76, $(M+H)^+$) |
| 11 | H | O$^t$Bu | Me | $^i$Bu | Me | Me | $^i$Bu | Bzl | 548 (100, $M^+$) |
| 12 | Bzl | O$^t$Bu | Pr | $^i$Bu | Me | Pr | $^i$Bu | Bzl | 695 (86, $(M+H)^+$) |
| 13 | Bzl | O$^t$Bu | Et | $^i$Bu | Me | Et | $^i$Bu | Bzl | 667 (70, $(M+H)^+$) |
| 14 | Bzl | O$^t$Bu | $^i$Pr | $^i$Bu | Me | $^i$Pr | $^i$Bu | Bzl | 695 (28, $(M+H)^+$) |
| 15 | H | O$^t$Bu | Pr | $^i$Bu | Me | Pr | $^i$Bu | Bzl | 605 (100, $(M+H)^+$) |
| 16 | Bzl | H | Et | $^i$Bu | Me | Et | $^i$Bu | Bzl | 611 (36, $(M+H)^+$) |
| 17 | Bzl | H | $^i$Pr | $^i$Bu | Me | $^i$Pr | $^i$Bu | Bzl | 639 (82, $M^+$) |
| 18 | H | O$^t$Bu | $^i$Pr | $^i$Bu | Me | $^i$Pr | $^i$Bu | Bzl | 605 (100, $M^+$) |
| 19 | Bzl | O$^t$Bu | Me | Bzl | Me | Me | Bzl | Bzl | 707 (15, $M^+$) |
| 20 | Bzl | H | Me | Bzl | Me | Me | Bzl | Bzl | 651 (64, $(M+H)^+$) |

| Bsp. Nr. | A | B | $R^1$ | $R^2$ | $R^3$ | $R^4$ | $R^5$ | $R^6$ | Massenspektroskop. Daten |
|---|---|---|---|---|---|---|---|---|---|
| 21 | H | H | Pr | $^i$Bu | Me | Pr | $^i$Bu | Bzl | |
| 22 | Bzl | O$^t$Bu | Me | $^i$Bu | Me | Me | $^i$Bu | 2-Cl-Bzl | 673 (100, $(M+H)^+$) |
| 23 | H | O$^t$Bu | Me | $^i$Bu | Me | Me | $^i$Bu | 2-Cl-Bzl | 583 (24, $(M+H)^+$) |
| 24 | Bzl | H | Me | $^i$Bu | Me | Me | $^i$Bu | 2-Cl-Bzl | 617 (36, $(M+H)^+$) |
| 25 | Bzl | O$^t$Bu | Me | Me | Me | Me | Me | Bzl | 555 (52, $(M+H)^+$) |
| 26 | H | O$^t$Bu | Me | Me | Me | Me | Me | Bzl | |
| 27 | Bzl | H | Me | Me | Me | Me | Me | Bzl | 499 (10, $(M+H)^+$) |
| 28 | Bzl | O$^t$Bu | Me | Pr | Me | Me | Pr | Bzl | 611 (100, $(M+H)^+$) |
| 29 | Bzl | O$^t$Bu | Me | $^s$Bu | $^i$Pr | Me | Bzl | $^i$Pr | |
| 30 | Bzl | O$^t$Bu | Me | $^s$Bu | $^i$Pr | Me | Bzl | $^i$Pr | 652 (8, $M^+$) |
| 31 | Bzl | O$^t$Bu | Me | $^i$Bu | Me | Me | $^i$Bu | Me | 562 (3, $M^+$) |
| 32 | H | O$^t$Bu | Me | $^i$Bu | Me | Me | $^i$Bu | Me | 472 (4, $M^+$) |
| 33 | Bzl | O$^t$Bu | Me | $^s$Bu | Me | Me | $^s$Bu | Me | 563 (1, $(M+H)^+$) |
| 34 | H | O$^t$Bu | Me | $^s$Bu | Me | Me | $^s$Bu | Me | 472 (1, $M^+$) |
| 35 | H | O$^t$Bu | Me | n-Bu | Me | Me | n-Bu | Me | 472 (1 , $M^+$) |
| 36 | Bzl | H | Me | $^s$Bu | Me | Me | $^s$Bu | Me | 506 (3 , $M^+$) |

| Bsp. Nr. | A | B | $R^1$ | $R^2$ | $R^3$ | $R^4$ | $R^5$ | $R^6$ | Massenspektroskop. Daten |
|---|---|---|---|---|---|---|---|---|---|
| 37 | Bzl | $O^tBu$ | Me | Pr | Me | Me | Pr | Me | 534 (2, $(M^+)$ |
| 38 | Bzl | $O^tBu$ | Me | $^iPr$ | Me | Me | $^iPr$ | Me | 534 (6, $M^+$) |
| 39 | Bzl | $O^tBu$ | Me | Bzl | Bzl | Me | Bzl | Me | 707 (5 , $(M+H)^+$) |
| 40 | Bzl | $O^tBu$ | Me | $^sBu$ | Bzl | Me | $^sBu$ | Me | 638 (5, $M^+$) |
| 41 | Bzl | $O^tBu$ | Me | $^iBu$ | H | Me | $^iBu$ | H | |
| 42 | H | $O^tBu$ | Me | $^iBu$ | Me | Me | $^iBu$ | H | |
| 43 | Bzl | H | Me | $^iBu$ | Me | Me | $^iBu$ | Me | |
| 44 | Bzl | H | Me | $^iBu$ | H | Me | $^iBu$ | H | |
| 45 | H | $O^tBu$ | Me | $^iBu$ | H | Me | $^iBu$ | H | |
| 46 | H | H | Me | $^iBu$ | Me | Me | $^iBu$ | Me | |
| 47 | H | H | Me | $^sBu$ | Me | Me | $^sBu$ | Me | |
| 48 | H | H | Me | Pr | Me | Me | Pr | Me | |

**Herstellung der Ausgangsstoffe der Formeln II und III**

**Beispiel II-1**

**N-Benzyl-N-methyl-L-leucyl-D-milchsäure-tert.-butylester**

Das Cäsiumsalz (77,7 g, 0,212 mol) im Bzl-L-MeLeu-OH wurde in Dimethylsulfoxid (530 ml) vorgelegt und bei Raumtemperatur mit 2-Chlor-propionsäure-tert.-butylester (34,76 g, 0,212 mol) versetzt. Es wurde 20 Stunden bei Raumtemperatur gerührt, in ges. Kochsalzlösung eingegossen und viermal mit Ethylacetat extrahiert. Die vereinigten organischen Extrakte wurden einmal mit wenig Wasser gewaschen, über Natriumsulfat getrocknet und eingeengt. Säulenchromatographie des Rückstandes an Kieselgel mit dem Laufmittel Cyclohexan-Ethylacetat = 60:1 ergab 63,5 g (82 %) Bzl-L-MeLeu-D-Lac-$O^tBu$.
Ei-MS m/z (%): 363 ($M^+$, 1), 190 (100).

**Beispiel III-1**

**N-Benzyl-N-methyl-L-leucyl-D-3-phenylmilchsäure-tert.-butylester**

Bzl-L-MeLeu-OH (50,0 g, 0,212 mol) wurde in Ethanol (1 000 ml) Wasser (100 ml) gelöst, mit einer 20 %igen Cäsiumcarbonatlösung (41,5 g, 0,127 Mol) in Wasser versetzt und 5 Stunden bei Raumtemperatur gerührt. Anschließend wurde eingeengt, zweimal mit je 250 ml DMF codestilliert und über Nacht bei 80°C im Hochvakuum getrocknet. Das Cäsiumsalz (77,7 g, 0,212 mol) wurde in Dimethylsulfoxid (530 ml) vorgelegt, bei Raumtemperatur mit 2-Chlor-3-phenylpropionsäure-tert.-butylester (51,0 g, 0,212 mol) versetzt und 20 Stunden bei Raumtemperatur gerührt.

Die Lösung wurde in gesättigte Kochsalzlösung eingegossen, viermal mit Ethylacetat extrahiert, über Natriumsulfat getrocknet und eingeengt. Säulenchromatographie des Rückstandes an Kieselgel mit dem Laufmittel Cyclohexan-Ethylacetat = 100:1 lieferte 26,6 g (29 %) sauberes Bzl-L-MeLeu-D-PheLac-$O^tBu$ sowie 48,9 g (52 %) einer mit Zimtsäure-tert.-butylester verunreinigten Mischfraktion.

Ei-MS m/z (%): 363 (1), 190 (100).

Analog können die in der nachstehenden Tabelle aufgeführten Verbindungen als L-D Stereoisomere hergestellt werden.

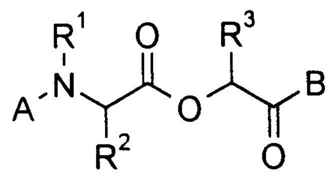

(II)

| Bsp.-Nr. | A | $R^1$ | $R^2$ | $R^3$ | B | Massen-spektroskopische Daten |
|---|---|---|---|---|---|---|
| II-2 | Bzl | Me | $-(CH_2)_2$-S-Me | Bzl | $O^tBu$ | 458 (100, $(M+H)^+$) |
| II-3 | Bzl | Me | $-(CH_2)_2$-S-Me | Me | $O^tBu$ | 382 (100, $(M+H)^+$) |
| II-4 | Bzl | Me | Pr | Me | OH | 294 (100, $(M+H)^+$) |
| II-5 | H | Me | Pr | Bzl | $O^tBu$ | 336 (100, $(M+H)^+$) |
| II-6 | Bzl | Me | Pr | Bzl | $O^tBu$ | 425 (2, $M^+$) |
| II-7 | Bzl | Me | Pr | Me | $O^tBu$ | 349 (5, $M^+$) |
| II-8 | H | Me | Me | Bzl | $O^tBu$ | 308 (100, $(M+H)^+$) |
| II-9 | Bzl | Me | Me | Bzl | $O^tBu$ | 398 (100, $(M+H)^+$) |
| II-10 | Bzl | Me | Me | Me | $O^tBu$ | 322 (100, $(M+H)^+$) |
| II-11 | Bzl | Me | Me | Me | OH | 265 (2, $M^+$) |
| II-12 | H | Me | $^iBu$ | p-Cl-Bzl | $O^tBu$ | 386 (100, $(M+H)^+$) |
| II-13 | H | Me | $^iBu$ | p-Cl-Bzl | $O^tBu$ | 384 (36, $(M+H)^+$) |
| II-14 | Bzl | Et | $^iBu$ | Me | OH | 322 (46, $(M+H)^+$) |
| II-15 | Bzl | $^iPr$ | $^iBu$ | Me | OH | 336 (100, $(M+H)^+$) |
| II-16 | Bzl | Pr | $^iBu$ | Me | OH | 336 (100, $(M+H)^+$) |
| II-17 | H | Et | $^iBu$ | Bzl | $O^tBu$ | 363 (0,5, $M^+$) |
| II-18 | H | $^iPr$ | $^iBu$ | Bzl | $O^tBu$ | |

| Bsp.-Nr. | A | $R^1$ | $R^2$ | $R^3$ | B | Massen-spektroskopische Daten |
|---|---|---|---|---|---|---|
| II-19 | H | Pr | $^i$Bu | Bzl | O$^t$Bu | 377 (0,4, $M^+$) |
| II-20 | Bzl | Et | $^i$Bu | Bzl | O$^t$Bu | 454 (100, $(M+H)^+$) |
| II-21 | Bzl | $^i$Pr | $^i$Bu | Bzl | O$^t$Bu | 467 (1 $M^+$) |
| II-22 | Bzl | Pr | $^i$Bu | Bzl | O$^t$Bu | 468 (100, $(M+H)^+$) |
| II-23 | Bzl | Et | $^i$Bu | Me | O$^t$Bu | 378 (100, $(M+H)^+$) |
| II-24 | Bzl | $^i$Pr | $^i$Bu | Me | O$^t$Bu | 392 (100, $(M+H)^+$) |
| II-25 | Bzl | Pr | $^i$Bu | Me | O$^t$Bu | 317 (46), 260 (42),139(100) |
| II-26 | H | Me | Bzl | Bzl | O$^t$Bu | 383 (6, $M^+$) |
| II-27 | Bzl | Me | Bzl | Bzl | O$^t$Bu | 400 (2), 382 (50), 326(36), 224(75) |
| II-28 | H | Me | $^s$Bu | Bzl | O$^t$Bu | 276(6), 236(15), 100(100) |
| II-29 | Bzl | Me | $^s$Bu | Me | OH | 307 (1, $M^+$) |
| II-30 | Bzl | Me | $^i$Pr | Me | OH | 293 (2, $M^+$) |
| II-31 | Bzl | Me | Bzl | Me | OH | 341 (1, $M^+$) |
| II-32 | H | Me | $^i$Pr | Bzl | O$^t$Bu | |
| II-33 | Bzl | Me | $^s$Bu | Me | O$^t$Bu | 363 (1, $M^+$) |
| II-34 | Bzl | Me | $^s$Bu | Bzl | O$^t$Bu | 439 (2, $M^+$) |

| Bsp.-Nr. | A | $R^1$ | $R^2$ | $R^3$ | B | Massen-spektroskopische Daten |
|---|---|---|---|---|---|---|
| II-35 | Bzl | Me | $^iPr$ | Me | $O^tBu$ | 349 (3, $M^+$) |
| II-36 | Bzl | Me | $^iPr$ | Bzl | $O^tBu$ | 425 (1, $M^+$) |
| II-37 | Bzl | Me | Bzl | Me | $O^tBu$ | 397 (0,5, $M^+$) |

## Patentansprüche

1. Verwendung von offenkettigen Tetradepsipeptiden der allgemeinen Formel (I)

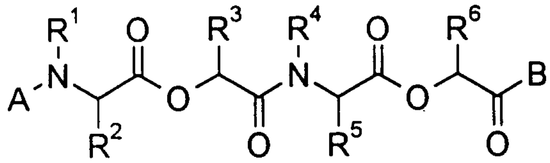

(I),

in welcher

A für Wasserstoff, Alkyl, Aralkyl oder einen Acylrest insbesondere der Formel -CO-$R^9$ steht, wobei

$R^9$ für geradkettiges oder verzweigtes Alkyl, Alkoxy, Aralkyl oder Aralkoxy mit bis zu 6 C-Atomen im Alkylteil steht,

$R^1$ und $R^4$ unabhängig voneinander für Wasserstoff, $C_1$-$C_8$-Alkyl, $C_3$-$C_6$-Cycloalkyl oder Aralkyl stehen,

$R^2$ und $R^5$ unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Aryl, Arylalkyl oder Hetarylmethyl, wobei als Substituenten genannt seien Halogen, Hydroxy, Alkyl, Alkoxy, Nitro, oder ein Rest -$NR^{10}R^{11}$, wobei $R^{10}$ und $R^{11}$ unabhängig voneinander für Wasserstoff oder Alkyl stehen oder $R^{10}$ und $R^{11}$ gemeinsam mit dem angrenzenden N-Atom für einen carbocyclischen 5-, 6-, 7- oder 8-gliedrigen Ring stehen, der gegebenenfalls durch O, S oder N unterbrochen sein kann und gegebenenfalls durch $C_1$-$C_4$-Alkyl substituiert ist, stehen,

$R^3$ und $R^6$ unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Alkylthioalkyl, Alkylsulfinylalkyl, Alkysulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl sowie gegebenenfalls substituiertes Aryl, Arylalkyl oder Hetarylmethyl, wobei als

Substituenten genannt seien Halogen, Hydroxy, Alkyl, Alkoxy,Nitro, oder ein Rest $-NR^{10}R^{11}$, wobei $R^{10}$ und $R^{11}$ unabhängig voneinander für Wasserstoff oder Alkyl stehen oder $R^{10}$ und $R^{11}$ gemeinsam mit dem angrenzenden N-Atom für einen carbocyclischen 5-, 6-, 7- oder 8-gliedrigen Ring stehen, der gegebenenfalls durch O, S oder N unterbrochen sein kann und gegebenenfalls durch $C_1$-$C_4$-Alkyl substituiert ist, stehen,

B — für Hydroxy, Alkoxy mit bis zu 4 Kohlenstoffatomen oder den Rest $NR^7R^8$ steht, wobei

$R^7$ und $R^8$ — für Wasserstoff, Alkyl, Aralkyl oder Aryl stehen,
sowie deren optische Isomere und Racemate,
zur Herstellung von endoparasitiziden Mitteln.

2. Endoparasitizide Mittel, gekennzeichnet durch einen Gehalt an mindestens einer Verbindung der Formel (I) gemäß Anspruch 1.

3. Verfahren zur Herstellung endoparasitizider Mittel, dadurch gekennzeichnet, daß man Verbindungen der Formel (I) gemäß Anspruch 1 mit Streckmitteln und/oder oberflächenaktiven Mitteln vermischt.

## Claims

1. Use of open-chain tetradepsipeptides of the general formula (I)

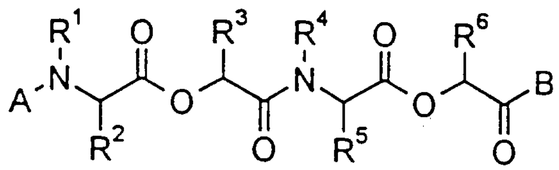

(I),

in which

A — represents hydrogen, alkyl, aralkyl or an acyl radical, in particular of the formula $-CO-R^9$, in which

$R^9$ — represents straight-chain or branched alkyl, alkoxy, aralkyl or aralkoxy having up to 6 C atoms in the alkyl moiety,

$R^1$ and $R^4$ — independently of one another represent hydrogen, $C_1$-$C_8$-alkyl, $C_3$-$C_6$-cycloalkyl or aralkyl,

$R^2$ and $R^5$ — independently of one another represent hydrogen, straight-chain or branched alkyl having up to 8 carbon atoms, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, guanidinoalkyl, which can optionally be substituted by one or two benzyloxycarbonyl radicals or by one, two, three or four alkyl radicals, or represent alkoxycarbonylaminoalkyl, 9-fluorenylmethoxycarbonyl(Fmoc)aminoalkyl, alkenyl, cycloalkyl, cycloalkylalkyl and optionally substituted aryl, arylalkyl or hetarylmethyl, substituents which may be mentioned being halogen, hydroxyl, alkyl, alkoxy, nitro or a radical $NR^{10}R^{11}$ in which $R^{10}$ and $R^{11}$ independently of one another represent hydrogen or alkyl or $R^{10}$ and $R^{11}$ together with the adjacent N atom represent a carbocyclic 5-, 6-, 7- or 8-membered ring which can optionally also be interrupted by O, S and N and which is optionally substituted by $C_1$-$C_4$-alkyl,

$R^3$ and $R^6$ — independently of one another represent hydrogen, straight-chain or branched alkyl having up to 8 carbon atoms, hydroxyalkyl, alkanoyloxyalkyl, alkoxyalkyl, aryloxyalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, alkoxycarbonylaminoalkyl, alkenyl, cycloalkyl, cycloalkylalkyl and optionally substituted aryl, arylalkyl or hetarylmethyl, substituents which may be mentioned being halogen, hydroxyl, alkyl, alkoxy, nitro or a radical $NR^{10}R^{11}$, in which $R^{10}$ and $R^{11}$ independently of one another represent hydrogen or alkyl or $R^{10}$ and $R^{11}$ together with the adjacent N atom represent a carbocyclic 5-, 6-, 7- or 8-membered ring which can optionally also be interrupted by O, S and N and which is optionally substituted by $C_1$-$C_4$-alkyl,

B represents hydroxyl, alkoxy having up to 4 carbon atoms or the radical $NR^7R^8$ in which

$R^7$ and $R^8$ represent hydrogen, alkyl, aralkyl or aryl,
and their optical isomers and racemates
for the preparation of endoparasiticidal compositions.

**2.** Endoparasiticidal compositions, characterized in that they contain at leat one compound of the formula (I) according to Claim 1.

**3.** Process for the preparation of endoparasiticidal compositions, characterized in that compounds of the formula (I) according to Claim 1 are mixed with extenders and/or surfactants.

## Revendications

**1.** Utilisation de tétradepsipeptides à chaîne ouverte de formule générale (I)

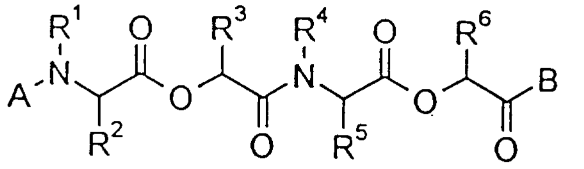

(I),

dans laquelle

A représente l'hydrogène, un reste alkyle, aralkyle ou un reste acyle notamment de formule $-CO-R^9$, dans laquelle $R^9$ est un groupe, linéaire ou ramifié, alkyle, alkoxy, aralkyle ou aralkoxy ayant jusqu'à 6 atomes de carbone dans la partie alkyle,

$R^1$ à $R^4$ représentent, indépendamment l'un de l'autre, l'hydrogène, un groupe alkyle en $C_1$ à $C_8$, cycloalkyle en $C_3$ à $C_6$ ou aralkyle,

$R^2$ et $R^5$ représentent, indépendamment l'un de l'autre, l'hydrogène, un groupe, linéaire ou ramifié, alkyle ayant jusqu'à 8 atomes de carbone, hydroxyalkyle, alcanoyloxyalkyle, alkoxyalkyle, aryloxyalkyle, mercaptoalkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, guanidinoalkyle, qui peut être substitué, le cas échéant par un ou deux restes benzyloxycarbonyle ou par un, deux, trois ou quatre restes alkyle, un groupe alkoxycarbonylaminoalkyle, 9-fluorénylméthoxycarbonyl(Fmoc)aminoalkyle, alcényle, cycloalkyle, cycloalkylalkyle ainsi qu'un groupe aryle, arylalkyle ou hétarylméthyle éventuellement substitué, et on mentionne alors comme substituants un halogène, un groupe hydroxy, alkyle, alkoxy, nitro ou un reste $-NR^{10}R^{11}$ dans lequel $R^{10}$ et $R^{11}$ représentent, indépendamment l'un de l'autre, l'hydrogène ou un groupe alkyle ou bien $R^{10}$ et $R^{11}$ forment conjointement avec l'atome d'azote adjacent un noyau carbocyclique pentagonal, hexagonal, heptagonal ou octogonal qui peut être interrompu, le cas échéant par O, S ou N et qui est substitué, le cas échéant par un reste alkyle en $C_1$ à $C_4$,

$R^3$ et $R^6$ représentent, indépendamment l'un de l'autre, l'hydrogène, un groupe linéaire ou ramifié alkyle ayant jusqu'à 8 atomes de carbone, hydroxyalkyle, alcanoyloxyalkyle, alkoxyalkyle, aryloxyalkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, alkoxycarbonylaminoalkyle, alcényle, cycloalkyle, cycloalkylalkyle ainsi qu'un groupe aryle, arylalkyle ou hétarylméthyle éventuellement substitué, et on mentionne alors comme substituants un halogène, un radical hydroxy, alkyle, alkoxy, nitro ou un reste $-NR^{10}R^{11}$, dans lequel $R^{10}$ et $R^{11}$ représentent, indépendamment l'un de l'autre, l'hydrogène ou un groupe alkyle ou bien $R^{10}$ et $R^{11}$ forment conjointement avec l'atome adjacent d'azote un noyau carbocyclique pentagonal, hexagonal, heptagonal ou octogonal qui peut être interrompu éventuellement par O, S ou N et qui est substitué, le cas échéant par un reste alkyle en $C_1$ à $C_4$,

B est un groupe hydroxy, alkoxy ayant jusqu'à 4 atomes de carbone ou le reste $NR^7R^8$, dans lequel

$R^7$ et $R^8$ représentent l'hydrogène, un groupe alkyle, aralkyle ou aryle,

ainsi que leurs isomères optiques et leurs racémates, pour la préparation de médicaments endoparasiticides.

2. Médicaments endoparasiticides, caractérisés par une teneur en au moins un composé de formule (I) suivant la revendication 1.

3. Procédé de préparation de compositions endoparasiticides, caractérisé en ce qu'on mélange des composés de formule (I) suivant la revendication 1 avec des diluants et/ou des agents tensio-actifs.